# FASCICULE DE BREVET EUROPEEN

(11) **EP 0 934 996 B1**
(45) Date de publication et mention de la délivrance du brevet: **06.05.2004**
(21) Numéro de dépôt: 99400228.5
(22) Date de dépôt: 02.02.1999
(51) Int. Cl.: C10G 25/00, C10G 25/05

(54) **Procédé de séparation chromatographique d'une charge C5-C8 ou d'une charge intermédiaire en trois effluents, respectivement riches en paraffines linéaires, monobranchées et multibranchées**
Verfahren zur chromatographischen Trennung von C5-C8 oder zwischenfraktionen in drei Ausflüsse, respektiv reich in geradkettigen, einfach - und mehrfach verzweigten Paraffinen
Process for the chromatographic separation of a C5-C8 or intermediate fraction into three effluents, respectively rich in linear, monobranched and multibranched parrafins

(30) Priorité: 04.02.1998 FR 9801390
(43) Date de publication de la demande: 11.08.1999
(73) Titulaire: Institut Français du Pétrole, 92500 Rueil Malmaison (FR)
(72) Inventeur: Ragil, Karine, 92500 Rueil Malmaison (FR); Jullian, Sophie, 92500 Rueil Malmaison (FR); Clause, Olivier, 78400 Chatou (FR)

(56) Documents cités:
- FR-A- 2 276 867
- FR-A- 2 496 486
- US-A- 4 426 292
- US-A- 5 055 634
- US-A- 5 107 052

## Description

L'invention concerne un procédé de séparation chromatographique permettant d'obtenir trois effluents respectivement riches en paraffines linéaires, en paraffines monobranchées et en paraffines dibranchées, tribranchées et éventuellement en composés naphténiques et/ou aromatiques, à partir de coupes C5-C8 ou de coupes intermédiaires (C5-C7, C6-C8, C7-C8, C6-C7, C7 ou C8), comprenant des hydrocarbures paraffiniques et éventuellement des hydrocarbures naphténiques et/ou aromatiques et, dans certains cas, oléfiniques.

Le procédé de séparation selon l'invention met en oeuvre une zone de séparation fonctionnant par adsorption. Le procédé est adapté à un fonctionnement en phase liquide ou en phase gazeuse. La zone de séparation selon l'invention contient au moins un adsorbant dont la mise en oeuvre est telle qu'il adsorbe préférentiellement les paraffines linéaires, dans une moindre mesure les paraffines monobranchées et enfin de façon minoritaire les paraffines multibranchées et les composés naphténiques et/ou aromatiques éventuellement présents. Le procédé de séparation selon l'invention est particulièrement utile lorsqu'il est couplé avec le procédé d'hydro-isomérisation tel que celui décrit dans la demande de brevet EP 0 922 750 A, puisqu'il permet un recyclage sélectif des paraffines linéaires et monobranchées, nécessaire pour les paraffines comportant au moins 7 atomes de carbone.

Dans le cas où la charge du procédé comprend la coupe C5, l'isopentane issu de cette coupe peut soit être séparé par le procédé selon l'invention avec les paraffines monobranchées ou les paraffines multibranchées selon la mise en oeuvre choisie, soit être retiré des flux traversant le procédé à l'aide d'au moins un déisopentaniseur disposé en amont et/ou en aval de l'unité de séparation.

### ART ANTÉRIEUR

La prise en compte de contraintes environnementales accrues entraîne la suppression des composés du plomb dans les essences, effective aux Etats-Unis et au Japon et en voie de généralisation en Europe. Dans un premier temps, les composés aromatiques, constituants principaux des essences de réformage et les isoparaffines produites par alkylation aliphatique ou isomérisation d'essences légères ont compensé la perte d'indice d'octane résultant de la suppression du plomb dans les essences. Par la suite, des composés oxygénés tels que le Méthyl Tertiobutyl Ether (MTBE) ou l'Ethyl Tertiobutyl Ether (ETBE) ont été introduits dans les carburants. Plus récemment, la toxicité reconnue de composés tels que les aromatiques, en particulier le benzène, les oléfines et les composés soufrés, ainsi que la volonté de diminuer la pression de vapeur des essences, ont entraîné aux Etats-Unis la production d'essences reformulées. Par exemple, les teneurs maximales en oléfines, composés aromatiques totaux et benzène dans les essences distribuées en Californie en 1996 sont respectivement de 6 % vol., 25 % vol. et 1 % vol. En Europe, les spécifications sont moins sévères ; néanmoins, la tendance prévisible est une réduction semblable des teneurs maximales en benzène, en composés aromatiques et en oléfines dans les essences produites et vendues.

Les « pools essence » comprennent plusieurs composants. Les composants majoritaires sont l'essence de réformage, qui comprend habituellement entre 60 et 80 % vol. de composés aromatiques, et les essences de craquage catalytique (FCC), qui contiennent typiquement 35 % vol. d'aromatiques, mais apportent de plus la majorité des composés oléfiniques et soufrés présents dans les essences. Les autres composants peuvent être les alkylats, sans composés aromatiques ni oléfiniques, les essences légères isomérisées ou non isomérisées, qui ne contiennent pas de composés insaturés, les composés oxygénés tels que le MTBE et des butanes. Dans la mesure où les teneurs maximales en aromatiques ne sont pas réduites en dessous de 30 ou 40 % vol., la contribution des réformats dans les « pools essence » restera importante, typiquement 40 % vol. Une sévérisation accrue de la teneur maximale admissible en composés aromatiques à 20 ou 25 % vol. entraînera une diminution de l'utilisation du réformage et, par voie de conséquence, la nécessité de valoriser les coupes C7-C10 de distillation directe par d'autres voies que le réformage. Leur valorisation par hydro-isomérisation est une des voies possibles, comme décrit dans la demande de brevet EP 0 922 750 A. Le procédé d'hydro-isomérisation conduit à la formation de composés multibranchés à partir des composés de faibles indices d'octane. Il ne peut être mis en oeuvre qu'à la condition de recycler les paraffines linéaires et monobranchées en C7-C10, puisque la réaction d'hydro-isomérisation est équilibrée et que ces paraffines de faibles indices d'octane ne peuvent pas être envoyées vers le « pool essence ». De plus, des conditions d'hydro-isomérisation différentes doivent être mises en oeuvre pour ces paraffines isomères afin d'éviter le craquage des paraffines les plus ramifiées. Ces deux points justifient la recherche de procédés de séparation permettant d'obtenir trois effluents distincts, respectivement un effluent riche en paraffines linéaires, un effluent riche en paraffines monobranchées et un effluent riche en paraffines multibranchées et éventuellement en composés naphténiques et/ou aromatiques.

L'utilisation de procédés de séparation par adsorption pour effectuer la séparation des paraffines linéaires, monobranchées et multibranchées a déjà fait l'objet de plusieurs brevets (par exemple US-A-4 717 784, 4 956 521 et 5 233 120, BE-A-891 522, FR-A-2 688 213, US-A-5 055 633, 4 367 364 et 4 517 402). Cependant, ces brevets ne s'appliquent d'une part qu'à la fraction légère C5-C6 et ne concernent de plus que la séparation de ces coupes de distillation en deux effluents, l'un de faible indice d'octane et l'autre de fort indice d'octane.

Ainsi, les brevets US-A-4 210 771 et 4 709 116 décrivent la séparation des paraffines linéaires à partir d'une coupe naphta C5-C6 en utilisant un adsorbant connu dans l'industrie sous le nom de zéolithe 5A au calcium. Le brevet US-A-4 367 364 décrit cette même séparation effectuée à l'aide de silicalite (US-A-4 061 724). De même, la demande de brevet FR-A-2 496 486 décrit un procédé de type chromatographique permettant d'effectuer cette même séparation en utilisant une zéolithe de type A, X, Y ou ZSM-5. Le procédé considéré permet de traiter toute charge gazeuse composée de plus de deux familles de constituants analogues et conduit à la production de deux effluents. Ce procédé est caractérisé en ce que, au cours d'une première étape, on injecte le mélange gazeux à traiter jusqu'à saturation de l'adsorbant, puis, après avoir cessé l'injection continue du mélange gazeux, on injecte périodiquement, dans une seconde étape, un gaz vecteur qui peut être de l'azote, de l'hélium ou de l'hydrogène ou un mélange de l'un de ces gaz inertes non adsorbables avec au maximum 40 % du gaz injecté dans la première étape. Ce procédé permet de recueillir successivement à la sortie de la colonne une fraction enrichie contenant la famille de constituants adsorbée purifiée et une fraction contenant les autres familles de constituants du mélange. Un tel procédé est particulièrement adapté au fractionnement en deux effluents d'une coupe C5-C6, comme le montrent les exemples de la demande de brevet FR-A-2 496 486. Dans ce cas, le procédé produit deux flux gazeux, le premier, riche en paraffines linéaires, et le second, riche en paraffines ramifiées (mono- et multibranchées), les deux effluents étant dilués dans l'éluant choisi parmi les trois gaz inertes non adsorbables suivants : azote, hélium ou hydrogène.

Les procédés de séparation décrits par ces différents brevets sont souvent couplés à un procédé d'isomérisation des paraffines linéaires puisque celles-ci possèdent un faible indice d'octane.

De la même façon, certains brevets (comme US-A-4 717 784 et 5 055 633) décrivent des procédés permettant d'effectuer la séparation des paraffines linéaires et des paraffines monobranchées à partir d'une coupe C5-C6. Ces paraffines linéaires et monobranchées constituent le pool de bas indice d'octane, alors que les paraffines multibranchées constituent le pool à haut indice d'octane. Dans ce cas, ces brevets soulignent l'intérêt d'utiliser des adsorbants tels que la ferriérite (US-A-4 804 802 et 4 717 784), les zéolithes ZSM-5 (US-A-3 702 886), ZSM-11 (US-A-4 108 881), ZSM-23 (US-A-4 076 842) et ZSM-35 (US-A-4 016 245) et la silicalite (US-A-5 055 633), puisque ces adsorbants adsorbent à la fois les paraffines linéaires et les paraffines monobranchées des coupes C5-C6 et excluent les paraffines de degrés de ramification plus élevés. Lors de la mise en oeuvre de tels adsorbants, l'isopentane est séparé de la charge et est produit dans le pool de faible indice d'octane, avec les paraffines linéaires et monobranchées, alors que ce composé possède un fort indice d'octane. Le brevet US-A-5 055 633 souligne en conséquence l'intérêt d'une mise en oeuvre permettant de produire l'isopentane avec le flux riche en composés multibranchés, composés naphténiques et/ou aromatiques à partir d'une charge C5-C6. Celle-ci contient au moins 10 % molaire d'isopentane, ainsi que des composés C7+ en quantités inférieures à 10 % molaire. Un tel procédé conduit à un flux secondaire riche en paraffines linéaires et monobranchées, qui peut être envoyé vers un réacteur d'isomérisation.

Dans les brevets cités plus haut, il n'est pas envisagé d'effectuer le fractionnement en trois effluents des coupes C5-C6 dans le cadre de leur isomérisation, ce pour deux raisons : d'une part, l'indice d'octane des paraffines monobranchées en C5-C6 est souvent jugé suffisant pour que ces composés soient envoyés vers le pool essence, auquel cas ces paraffines sont séparées avec les paraffines multibranchées. D'autre part, lorsque les paraffines linéaires et les paraffines monobranchées sont recyclées vers l'isomérisation, il n'est pas utile de les séparer, puisque ces composés peuvent être isomérisés dans les mêmes conditions opératoires, contrairement aux coupes plus lourdes, telles que celles concernées par la présente invention.

Le brevet US-A-5 055 634 est le seul à décrire un procédé pouvant conduire à trois flux respectivement riches en paraffines linéaires, monobranchées et multibranchées à partir d'une coupe légère C5-C6, mais son intérêt principal, tout comme celui du procédé du brevet US-A-5 055 633, réside dans la possibilité de séparer et de produire l'isopentane avec le flux riche en paraffines multibranchées. La charge d'un tel procédé contient au moins 10 % molaire d'isopentane. Elle est centrée sur C5-C6 et peut parfois contenir de faibles quantités de paraffines comportant sept atomes de carbone ou plus. En conséquence, le procédé décrit dans ce brevet s'applique pour des teneurs en ces composés C7+ inférieures à 10 % molaire.

Le procédé décrit dans le brevet US-A-5 055 634 comprend la mise en oeuvre de deux unités disposées en série. La charge arrive dans la première unité, qui contient un adsorbant capable de retenir sélectivement les paraffines linéaires. L'effluent de cette unité est en conséquence constitué de paraffines mono- et multibranchées. Cet effluent « dénormalisé » est alors introduit dans la seconde unité remplie d'un adsorbant capable de retenir préférentiellement les paraffines monobranchées à l'exception de l'isopentane, qui est produit avec les paraffines multibranchées. Ce brevet indique que la régénération des deux unités s'effectue à l'aide d'un gaz non adsorbable, comme par exemple l'hydrogène. Celui-ci. parcourt tout d'abord la seconde unité et permet de désorber les paraffines monobranchées. Au moins une partie de ce flux est alors envoyé vers la première unité et permet de désorber les paraffines linéaires qui y sont contenues. La régénération ainsi effectuée conduit à mélanger une partie des paraffines monobranchées aux paraffines linéaires précédemment séparées à l'exception de l'isopentane, qui est récupéré avec les composés de fort indice d'octane dans le flux de production. Dans une version préférée de ce procédé, l'ensemble du flux de désorption sortant de la deuxième unité parcourt la première afin de minimiser la quantité de gaz non adsorbable nécessaire pour régénérer l'ensemble des deux unités. Dans ce dernier cas, le procédé ne produit que deux flux, le premier riche en paraffines multibranchées, composés naphténiques, aromatiques et isopentane, le second riche en paraffines linéaires et monobranchées. Une telle séparation peut alors être effectuée à l'aide d'un seul adsorbeur contenant deux types d'adsorbants conformément à l'exemple donné dans ce brevet US-A-5 055 634.

Les techniques de séparation par adsorption préconisées par ces différents brevets afin de valoriser les coupes C5-C6 sont celles connues par l'homme de l'art. Ainsi, le procédé de séparation par adsorption peut être de type PSA (Pressure Swing Adsorption), TSA (Temperature Swing Adsorption), chromatographique (chromatographie d'élution ou contre-courant simulé par exemple) ou résulter d'une combinaison de ces techniques. Ces procédés ont tous en commun de mettre en contact un mélange liquide ou gazeux avec un lit fixe d'adsorbant afin d'éliminer certains constituants du mélange qui peuvent être adsorbés.

La chromatographie ou fractionnement isobare, en phase gazeuse ou en phase liquide est une technique de séparation très efficace grâce à la mise en oeuvre d'un très grand nombre d'étages théoriques. Elle permet ainsi de tirer parti de sélectivités d'adsorption relativement faibles et de réaliser des séparations difficiles. Les procédés N-ISELF® de Elf Aquitaine (« Separating Paraffin Isomers Using Chromatography », Chemical Engineering, 18, p 92-95 (1981)) pour la séparation des n/isoparaffines en C5-C6 et ASAHI (Seko M., Miyake J., Inada K., lnd. Eng. Chem. Prod. Res. Develop.,1979, 18, 263) pour la séparation du paraxylène et de l'éthylbenzène d'une coupe C8 aromatique, présentent ce type de mise en oeuvre pour effectuer des séparations en deux flux. Ces techniques de séparation isobare sont connues par l'homme de l'art. Une description plus approfondie peut être trouvée dans l'ouvrage plus général de Yang (« Gas Separation by Adsorption Processes », Butterworth Publishers, US, 1987).

### RÉSUMÉ DE L'INVENTION

L'invention concerne un procédé de séparation chromatographique permettant d'obtenir trois effluents respectivement riches en paraffines linéaires, en paraffines monobranchées et en paraffines dibranchées, tribranchées et éventuellement en composés naphténiques et/ou aromatiques, à partir de coupes légères C5-C8 ou de coupes intermédiaires, telles que C5-C7, C6-C8, C7-C8, C6-C7, C7 ou C8, comprenant des hydrocarbures paraffiniques et éventuellement naphténiques, aromatiques et, dans certains cas, oléfiniques.

Le procédé de séparation selon l'invention comprend une zone de séparation et est adapté à un fonctionnement en phase liquide ou en phase gazeuse. Un tel procédé de séparation est particulièrement utile lorsqu'il est couplé avec un procédé d'hydro-isomérisation tel que décrit dans la demande de brevet EP0 922 750 A En effet, le procédé décrit nécessite de recycler à la fois les paraffines linéaires (nCx, x = 5 à 8) et les paraffines monobranchées (monoCx), puisque les paraffines en C7-C8 ont de faibles indices d'octane (voir Tableau 1 ci-dessous). De plus, des conditions d'hydroisomérisation différentes doivent être mises en oeuvre pour ces deux types d'isomères pour éviter le craquage des paraffines les plus ramifiées. Ces deux points justifient la recherche de procédé de séparation permettant d'obtenir trois effluents distincts respectivement riches en paraffines linéaires nCx, en paraffines monobranchées monoCx et en paraffines multibranchées (diCx ou triCx), composés naphténiques et/ou aromatiques.

**Tableau 1**

| Paraffine | nC7 | monoC6 | diC5 | tri C4 | nC8 | monoC7 | di C6 | tri C5 |
|---|---|---|---|---|---|---|---|---|
| RON | 0 | 42-52 | 80-93 | 112 | < 0 | 21-27 | 55-76 | 100-109 |
| MON | 0 | 23-39 | 84-95 | 101 | < 0 | 23-39 | 56-82 | 96-100 |

D'une manière générale, le procédé selon l'invention est caractérisé en ce que la charge est injectée périodiquement dans une zone de séparation chromatographique en alternance avec un éluant EP 0 922 750 A non adsorbable. La zone de séparation chromatographique selon l'invention contient au moins un adsorbant dont la mise en oeuvre est telle qu'il adsorbe préférentiellement les paraffines linéaires, dans une moindre mesure les paraffines monobranchées et enfin de façon minoritaire les paraffines multibranchées et les composés naphténiques et aromatiques. L'éluant est choisi de manière à permettre la désorption des différents composés adsorbés de la charge. Ce procédé permet de recueillir successivement à la sortie de l'adsorbeur une première fraction enrichie en paraffines multibranchées et éventuellement en composés naphténiques et/ou aromatiques, une seconde fraction enrichie en paraffines monobranchées et enfin une dernière fraction enrichie en paraffines linéaires.

### DESCRIPTION DÉTAILLÉE DE L'INVENTION

La charge traitée dans le procédé selon l'invention provient de la coupe C5-C8 ou de toutes coupes intermédiaires (comme par exemple C5-C7, C6-C8, C6-C7, C7-C8, C7 ou C8) issues de la distillation atmosphérique, d'une unité de réformage (réformat léger) ou d'une unité de conversion (naphta d'hydrocracking par exemple). Dans la suite du texte, cet ensemble de charges possibles sera désigné par les termes « coupe C5-C8 et coupes intermédiaires ».

En général, la charge traitée est composée principalement de paraffines linéaires, monobranchées et multibranchées, de composés naphténiques, tels que les diméthylcyclopentanes, de composés aromatiques, tels que le benzène et/ou le toluène, et éventuellement de composés oléfiniques. On regroupera sous le vocable « paraffines multibranchées », toutes les paraffines dont le degré de ramification est égal ou supérieur à deux.

La charge peut notamment contenir du normal pentane, du 2-méthylbutane, du néopentane, du normal hexane, du 2-méthylpentane, du 3-méthylpentane, du 2,2-diméthylbutane, du 2,3 diméthylbutane, du normal heptane, du 2-méthylhexane, du 3-méthylhexane, du 2,2-diméthylpentane, du 3,3-diméthylpentane, du 2,3-diméthylpentane, du 2,4-diméthylpentane, du 2,2,3-triméthylbutane, du normal octane, du 2-méthylheptane, du 3-méthylheptane, du 4-méthylheptane, du 2,2-diméthylhexane, du 3,3-diméthylhexane, du 2,3-diméthylhexane, du 3,4-diméthylhexane, du 2,4-diméthylhexane, du 2,5-diméthylhexane, du 2,2,3-triméthylpentane, du 2,3,3-triméthylpentane et du 2,3,4-triméthylpentane. Dans la mesure où la charge vient des coupes C5-C8 ou de coupes intermédiaires (comme par exemple C5-C7, C6-C8, C6-C7, C7-C8, C7, C8) obtenues après distillation atmosphérique, elle peut de plus contenir des alcanes cycliques, tels que les diméthylcyclopentanes, des hydrocarbures aromatiques (tels que benzène, toluène et/ou xylènes), ainsi que des hydrocarbures en C9+ (c'est-à-dire des hydrocarbures contenant au moins 9 atomes de carbone) en quantité moindre. Les charges C5-C8 et celles constituées de coupes intermédiaires provenant d'un réformat peuvent contenir en outre des hydrocarbures oléfiniques, en particulier lorsque les unités de réformage. dont elles sont issues sont opérées à basse pression.

La teneur en paraffines (P) dépend essentiellement de l'origine de la charge, c'est-à-dire de son caractère paraffinique ou de son caractère naphténique et aromatique, ce dernier étant parfois mesuré par le paramètre (N+A) (somme de la teneur en naphtènes (N) et de la teneur en aromatiques (A)), ainsi que de son point initial de distillation, c'est-à-dire de la teneur en C5 et C6 dans la charge. Dans les naphtas d'hydrocraquage, riches en composés naphténiques, ou les réformats légers, riches en composés aromatiques, la teneur en paraffines dans la charge sera en général faible, de l'ordre de 30 % en masse. Dans les coupes C5-C8 ou dans les coupes intermédiaires de distillation directe, la teneur en paraffines varie entre 30 et 80 % en masse, avec une valeur moyenne de 55-60 % en masse.

La charge riche en paraffines comprenant entre 5 et 8 atomes de carbone est en général de faible indice d'octane et le procédé selon l'invention consiste à la fractionner en trois effluents distincts d'indice d'octane moteur et recherche croissants, respectivement riches en paraffines linéaires, en paraffines monobranchées et en paraffines dibranchées, tribranchées et éventuellement en composés naphténiques et/ou aromatiques.

Le fractionnement s'effectue dans une zone de séparation contenant un ou plusieurs adsorbants, à l'aide d'un éluant non adsorbable. Le procédé suivant l'invention est adapté à un fonctionnement en phase liquide ou gazeuse. De plus, généralement plusieurs unités de séparation (par exemple de deux à quinze) sont utilisées en parallèle et alternativement pour conduire à un procédé fonctionnant de façon continue, bien qu'un procédé chromatographique tel que celui de l'invention est discontinu par nature.

Le procédé selon l'invention est caractérisé en ce que la charge est injectée périodiquement dans une zone de séparation (ou adsorbeur) en alternance avec un éluant non adsorbable. La zone de séparation selon l'invention contient au moins un adsorbant dont la mise en oeuvre est telle qu'elle adsorbe préférentiellement les paraffines linéaires, dans une moindre mesure les paraffines monobranchées et enfin de façon minoritaire les paraffines multibranchées et les composés naphténiques et aromatiques. Il se crée en conséquence au sein de l'adsorbeur, lors de l'injection de la charge, des fronts d'adsorption en ces différents constituants. Un éluant non adsorbable est alors injecté dans l'adsorbeur. Il est choisi de manière à permettre la désorption des différents composés adsorbés de la charge, qui traversent alors l'adsorbeur suivant des fronts de désorption distincts. Ce procédé permet de recueillir successivement à la sortie de l'adsorbeur une première fraction enrichie en paraffines multibranchées, et éventuellement composés naphténiques et/ou aromatiques, une seconde fraction enrichie en paraffines monobranchées et enfin une dernière fraction enrichie en paraffines linéaires.

Dans le cas où la charge comprend des composés oléfiniques, leur fractionnement dans la séparation chromatographique se fait selon leur degré de ramification, comme pour les composés paraffiniques.

L'éluant mis en jeu dans le procédé de l'invention est non adsorbable, auquel cas la régénération de l'adsorbant est assurée par abaissement des concentrations des espèces adsorbées. Dans ce cas, on peut utiliser par exemple l'hydrogène, l'azote, l'hélium, ou encore tout composé exclu géométriquement des pores de l'adsorbant.

Lorsque la charge contient la coupe C5, l'isopentane provenant de cette coupe peut soit être séparé par le procédé selon l'invention dans l'effluent contenant les paraffines monobranchées ou dans celui contenant les paraffines multibranchées, selon la mise en oeuvre choisie, soit être retiré des flux traversant le procédé à l'aide d'au moins un déisopentaniseur disposé en amont et/ou en aval de l'unité de séparation.

Plus généralement, il peut être intéressant de prélever une ou plusieurs fractions légères de la charge en amont de l'adsorbeur ou une ou plusieurs fractions légères des flux obtenus en sortie de l'adsorbeur enrichis soit en paraffines linéaires, soit en paraffines monobranchées.

La zone de séparation chromatographique est remplie d'un adsorbant naturel ou synthétique capable de séparer les paraffines linéaires, les paraffines monobranchées et les paraffines multibranchées, composés naphténiques et/ou aromatiques sur la base des différences entre les propriétés géométriques, diffusionnelles ou thermodynamiques des adsorbats pour les adsorbants considérés. Il existe un grand nombre de matériaux adsorbants permettant d'effectuer ce type de séparation. Parmi eux, se trouvent les tamis moléculaires au carbone, les argiles activées, le silica gel, l'alumine activée et les tamis moléculaires cristallins. Ces derniers ont une taille de pore uniforme et sont pour cette raison particulièrement adaptés à ce type de séparation. Ces tamis moléculaires incluent notamment les différentes formes de silicoaluminophosphates et d'aluminophosphates décrits dans les brevets US-A-4 444 871, 4 310 440 et 4 567 027, aussi bien que les tamis moléculaires zéolithiques. Ceux-ci sous leur forme calcinée peuvent être représentés par la formule chimique :

M_{2/n} O : Al₂O₃ : x SiO₂ : y H₂O

où M est un cation, x est compris entre 2 et l'infini, y a une valeur comprise entre 2 et 10 et n est la valence du cation.

On préférera pour le procédé selon l'invention des tamis moléculaires microporeux ayant un diamètre de pore effectif légèrement supérieur à 5 Å (1Å = 10⁻¹⁰ m). Le terme « diamètre de pore » est conventionnel pour l'homme du métier. Il est utilisé pour définir de façon fonctionnelle la taille d'un pore en terme de taille de molécule capable d'entrer dans ce pore. Il ne désigne pas la dimension réelle du pore, car celle-ci est souvent difficile à déterminer, puisque souvent de forme irrégulière (c'est-à-dire non circulaire). D.W. Breck fournit une discussion sur le diamètre de pore effectif dans son ouvrage intitulé « Zeolite Molecular Sieves » (John Wiley and Sons, New York, 1974), pages 633 à 641. Parmi les tamis moléculaires préférés pour l'invention, se trouvent ceux possédant des pores de section elliptique de dimensions comprises entre 5,0 et 5,5 Å suivant le petit axe et environ 5,5 à 6,0 Å suivant le grand axe. Un adsorbant présentant ces caractéristiques et donc particulièrement adapté à la présente invention est la silicalite. Le terme silicalite inclut ici à la fois les silicopolymorphes décrits dans le brevet US-A-4 061 724 et aussi la silicalite-F décrite dans le brevet US-A-4 073 865. D'autres adsorbants présentant ces mêmes caractéristiques et qui sont en conséquence particulièrement adaptés à la présente application sont la ZSM-5, la ZSM-11, la ZSM-23 (US-A-4 076 842), la ZSM-35 (US-A-4 016 245) ou la ZSM-48, ainsi que de nombreux autres aluminosilicates cristallins analogues. La ZSM-5 et la ZSM-11 sont décrites dans les brevets US-A-3 702 886, RE 29 948 et US-A-3 709 979. La teneur en silice de ces adsorbants peut être variable. Les adsorbants les plus adaptés à ce type de séparation sont ceux qui présentent des teneurs en silice élevées. Le rapport molaire Si/Al doit être de préférence au moins égal à 10 et de manière préférée supérieur à 100. Un autre type d'adsorbant particulièrement adapté à la présente application possède des pores de section elliptique de dimensions comprises entre 4,5 et 5,5 Å. Ce type d'adsorbant a été caractérisé par exemple dans le brevet US-A-4 717 748 comme étant un tectosilicate possédant des pores de taille intermédiaire entre celle des pores du tamis au calcium 5A et celle des pores de la ZSM-5. Les adsorbants préférés incluent la ZSM-5, la ZSM-11 et la ZSM-23, ainsi que la ferriérite (décrite dans les brevets US-A-4 016 425 et 4 251 499).

Ces différents adsorbants ont des tailles de pore telles que chacun des isomères des coupes C5-C8 ou des coupes intermédiaires peut être adsorbé. La cinétique de diffusion de ces isomères est cependant suffisamment différente pour être mise à profit. Dans certaines conditions de mise en oeuvre, ces tamis moléculaires permettent d'effectuer la séparation en trois effluents décrite dans la présente invention. Les détails de l'adsorption des paraffines linéaires, monobranchées, multibranchées, des composés naphténiques et aromatiques sur chacun de ces tamis sont connus de l'homme de l'art et ce processus ne fera donc pas l'objet ici d'une plus ample description.

Il peut aussi être intéressant dans la présente invention de mélanger aux adsorbants cités ci-dessus des zéolithes de type 5A, telles que celles décrites dans le brevet US-A-2 882 243. Dans la plupart de leurs formes cationiques échangées, notamment sous la forme calcium, ces zéolithes présentent un diamètre de pore de l'ordre de 5 Å et possèdent de fortes capacités pour adsorber les paraffines linéaires. Mélangées avec les zéolithes précédemment citées, elles peuvent permettre d'accentuer la séparation des fronts d'élution et donc d'obtenir une meilleure pureté de chacun des flux enrichis obtenus.

Les conditions opératoires de l'unité de séparation dépendent du ou des adsorbants considérés, ainsi que du degré de pureté désiré pour chacun des flux. Elles sont comprises entre 50 et 450 °C pour la température et de 0,01 à 7 MPa pour la pression. Plus précisément, si la séparation est effectuée en phase liquide, les conditions de séparation sont : 50 à 250 °C pour la température et 0,1 à 7 MPa pour la pression. Si ladite séparation est effectuée en phase gazeuse, ces conditions sont : 150 à 450 °C pour la température et 0,01 à 7 MPa pour la pression. La durée des injections périodiques du mélange à traiter est comprise entre 1 et 200 secondes. La durée de chaque cycle est comprise entre 10 et 2000 secondes et le rapport durée d'injection/durée de cycle est inférieur à 0,5.

Les exemples qui suivent illustrent une séparation chromatographique utilisant l'isopentane comme éluant adsorbable. Ce mode d'élution correspond à celui décrit dans la demande de brevet EP 0 922 750 et n'est pas l'objet de la présente invention.

### EXEMPLE 1

On donne ci-après un exemple dans lequel la séparation chromatographique est effectuée par adsorption en phase gazeuse à partir d'une coupe de distillation directe C5-C8 comprenant des hydrocarbures paraffiniques, naphténiques et aromatiques.

La charge fraîche du procédé possède la composition indiquée dans le Tableau 2 et en conséquence un indice d'octane recherche de 65 et un indice d'octane moteur de 63,5.

La charge fraîche est envoyée avec un débit de 273,5 kg/h vers un déisopentaniseur. La fraction légère recueillie en tête de ce déisopentaniseur n°1 a la composition indiquée dans le Tableau 3, un indice d'octane recherche de 92,4 et un débit de 28,5 kg/h. Cette fraction est utilisée par la suite comme éluant dans l'unité de séparation.

**Tableau 2**

| Composants | masse % |
|---|---|
| ic4 | 0,02 |
| nC4 | 0,91 |
| nC5 | 15,23 |
| iC5 | 9,50 |
| cyclopentane | 0,73 |
| nC6 | 15,80 |
| monobranchés en C6 | 12,61 |
| dibranchés en C6 | 5,30 |
| cyclohexane | 2,34 |
| méthylcyclopentane | 3,27 |
| nC7 | 7,45 |
| monobranchés en C7 | 3,95 |
| dibranchés en C7 | 1,06 |
| tribranchés en C7 | 1,20 |
| diméthylcycloC5 | 4,58 |
| méthylcycloC6 | 3,79 |
| nC8 | 1,12 |
| monobranchés C8 | 0,93 |
| dibranchés C8 | 0,77 |
| tribranchés C8 | 0,28 |
| triméthylcycloC5 | 4,03 |
| éthylbenzene | 0,99 |
| toluene | 3,73 |
| Benzène | 0,41 |

**Tableau 3**

| Composant | masse % |
|---|---|
| nC4 | 7 |
| iC4 | 0,15 |
| iC5 | 92,85 |

La charge déisopentanisée (débit 245 kg/h), préalablement réchauffée et vaporisée à 250 °C et à une pression de 1,0 MPa, arrive dans l'unité de séparation. Cette unité comprend 9 adsorbeurs qui sont des cylindres de 0,3 m de diamètre interne et de 4 m de hauteur, contenant chacun 226 kg de silicalite, mis sous forme de billes de 1,2 mm de diamètre. La charge et l'éluant alimentent l'unité de séparation sous contrôle de débit et les effluents sont recueillis sous contrôle de pression. Chaque unité de séparation subit cycliquement les étapes décrites ci-après.

### 1 - Injection de la charge déisopentanisée :

La charge déisopentanisée (245,05 kg/h) pénètre dans le lit qui contient du gaz éluant. En raison de leur cinétique de diffusion et de leur propriétés thermodynamiques d'adsorption différentes au sein de l'adsorbant, les composés sont retenus suivant des fronts d'adsorption distincts. Les paraffines linéaires sont les plus retenues, alors que les paraffines multibranchées, les composés naphténiques et aromatiques ne le sont que très peu dans ces conditions de fonctionnement.

### 2 - Injection de l'éluant :

L'isopentane est alors envoyé à co-courant de la première étape avec le même débit que la charge. L'isopentane permet de désorber les différents composés adsorbés de façon sélective. Son introduction conduit à la formation de plusieurs flux d'élution au sein de l'unité de séparation.

On recueille ainsi en sortie de l'adsorbeur trois flux enrichis respectivement en paraffines dibranchées, composés naphténiques et aromatiques, en paraffines monobranchées et en paraffines linéaires.

Le fonctionnement décrit ci-dessus est celui d'un des adsorbeurs. Les neuf adsorbeurs qui forment l'unité de séparation fonctionnent de la même manière mais de façon décalée, afin de conduire à la production continue des trois effluents.

L'injection de la charge est effectuée pendant 50 secondes, celle de l'éluant pendant 450 secondes.

Le flux enrichi en paraffines multibranchées, en composés naphténiques et aromatiques, est recueilli pendant 166 secondes en sortie d'adsorbeur. L'indice d'octane recherche de cette fraction est de 91,9. Ce flux contient 89 % d'isopentane et 0,26 % de paraffines linéaires et 0,97 % de paraffines monobranchées.

Le flux enrichi en paraffines monobranchées est ensuite recueilli pendant 99 secondes en sortie d'adsorbeur. L'indice d'octane recherche de cette fraction est de 89,7. Ce flux contient 90 % d'isopentane, 1,48 % de paraffines linéaires et 1,78 % de paraffines dibranchées, composés naphténiques et composés aromatiques.

Enfin, le flux enrichi en paraffines linéaires est recueilli pendant 235 secondes en sortie d'adsorbeur. L'indice d'octane recherche de cette fraction est de 86,8. Ce flux contient 91 % d'isopentane, 0,68 % de paraffines monobranchées et 0,08 % de paraffines dibranchées, composés naphténiques et composés aromatiques.

Globalement, le procédé suivant l'invention conduit à la production de trois effluents respectivement riches en paraffines linéaires, en paraffines monobranchées et en paraffines multibranchées, composés naphténiques et/ou composés aromatiques, à partir d'une coupe de distillation directe C5-C8 comprenant des hydrocarbures paraffiniques, naphténiques et/ou aromatiques.

On rappelle que, dans le cadre de l'invention « Essences à haut indice d'octane et leur production par un procédé associant hydro-isomérisation et séparation », qui a fait l'objet du dépôt d'une demande de brevet français au nom de la Demanderesse le 25 novembre 1997, les trois flux enrichis issus de la séparation peuvent être envoyés vers des unités d'hydro-isomérisation, pour les deux flux de plus bas indice d'octane, et vers le « pool essence », pour le flux de fort indice d'octane. Les flux en sortie des sections hydro-isomérisation peuvent alors être recyclés vers le procédé décrit ci-dessus. Il peut être aussi intéressant, par exemple dans le but de limiter le volume des sections d'hydro-isomérisation, de déisopentaniser le flux enrichi en paraffines monobranchées et/ou le flux enrichi en paraffines linéaires. Le flux enrichi en paraffines multibranchées et éventuellement en composés naphténiques et/ou en composés aromatiques peut également être envoyé dans un déisopentaniseur.

L'exemple qui suit décrit le cas où les trois flux issus de la séparation chromatographique sont traités par des déisopentaniseurs.

### EXEMPLE 2

On répète l'Exemple 1, mais on traite les trois flux enrichis obtenus de la manière décrite ci-après.

Ces trois flux sont respectivement envoyés vers trois déisopentaniseurs distincts (respectivement déisopentaniseur n°2 pour le flux enrichi en paraffines multibranchées, déisopentaniseur n°3 pour le flux enrichi en paraffines monobranchées et déisopentaniseur n°4 pour le flux enrichi en paraffines linéaires), afin d'extraire !'isopentane de ces trois flux et de le recycler comme gaz éluant vers l'unité de séparation. Les compositions des trois flux issus des trois déisopentaniseurs, qui sont respectivement riches en paraffines multibranchées, composés naphténiques et composés aromatiques, en paraffines monobranchées et en paraffines linéaires, sont données dans le Tableau 4.

Le procédé tel qu'illustré dans cet exemple requiert le recyclage en boucle fermée d'une certaine quantité d'isopentane entre les déisopentaniseurs n°2, n°3 et n°4 et les 9 unités de séparation chromatographique. Le débit de ce gaz éluant peut être ajusté en fonction des spécifications des unités de séparation. Une partie de ce gaz éluant circulant en boucle fermée peut être prélevée. Cette partie correspond à la quantité de la fraction légère prélevée par le déisopentaniseur n°1 de la charge fraîche.

**Tableau 4**

| Composition du flux enrichi en paraffines linéaires après passage dans le déisopentaniseu r n°2 | (% masse) | Composition du flux enrichi en paraffines monobranchées après passage dans le déisopentaniseur n°3 | (% masse) | Composition du flux enrichi en paraffines multibranchées après passage dans le déisopentaniseur n°4 | (% masse) |
|---|---|---|---|---|---|
| nC5 | 35 | isopentane | 0,5 | multibranchés en C6 | 15,9 |
| nC6 | 36 | monobranchés en C6 | 48 | multibranchés en C7 | 7,6 |
| nC7 | 17 | monobranchés en C7 | 15 | multibranchés en C8 | 3,1 |
| nC8 | 2,5 | monobranchés en C8 | 3,5 | composés aromatiques et naphtènes | 59,1 |
| composés monobranchés | 8,7 | composés linéaires | 15 | composés monobranchés | 11,7 |
| composés multibranchés | 0,8 | composés multibranchés | 18 | composés linéaires | 2,6 |
| RON | 36,5 | | 67,3 | | 92.4 |

## Revendications

1. Procédé de séparation de paraffines linéaires, monobranchés et multibranchés à partir d'une charge constituée d'une coupe légère comprenant des hydrocarbures paraffiniques en C5-C8, C5-C7, C6-C8, C7-C8, C6-C7, C7 ou C8, **caractérisé en ce que** l'on fait passer alternativement et en co-courant, dans une zone de séparation unique contenant au moins un lit d'adsorbant, la totalité de ladite charge et un éluant, ledit adsorbant étant choisi parmi les adsorbants naturels ou synthétiques capables de séparer les paraffines linéaires, les paraffines monobranchées et les paraffines multibranchées et ledit éluant consistant en un composé non adsorbable par l'adsorbant de la zone de séparation et **en ce que** l'on sépare les paraffines linéaires, les paraffines monobranchées et les paraffines multibranchées sous forme de trois flux d'élution sortant successivement de ladite zone de séparation.

2. Procédé selon la revendication 1, **caractérisé en ce que** la charge comprend en outre des hydrocarbures naphténiques et/ou aromatiques, qui sont séparés avec l'effluent riche en paraffines multibranchées.

3. Procédé selon la revendication 1 ou 2, **caractérisé en ce que** la charge comprend la coupe C5 et l'isopentane issu de cette coupe est séparé avec les paraffines monobranchées.

4. Procédé selon l'une des revendications 1 à 3, **caractérisé en ce que**, la charge comprend la coupe C5, ledit procédé comprend en outre une étape préliminaire dans laquelle on sépare l'isopentane contenu dans cette coupe en amont de la zone de séparation.

5. Procédé selon l'une des revendications 1 à 4, **caractérisé en ce que** la charge comprend la coupe C5 et l'isopentane contenu dans cette coupe est retiré sur au moins un des effluents sortant du procédé.

6. Procédé selon la revendication 5, **caractérisé en ce que** la charge comprend la coupe C5 et l'isopentane contenu dans cette coupe est retiré sur l'effluent riche en paraffines monobranchées sortant du procédé.

7. Procédé selon l'une des revendications 1 à 6, **caractérisé en ce que** l'on utilise au moins un adsorbant choisi dans le groupe formé par la silicalite, la zéolithe ZSM-5, la zéolithe ZSM-11, la zéolithe ZSM-23 et la ferriérite.

8. Procédé selon la revendication 7, **caractérisé en ce que** ledit adsorbant comprend en outre une zéolithe 5A.

9. Procédé selon l'une des revendications 1 à 8, **caractérisé en ce que** la séparation est effectuée en phase liquide.

10. Procédé selon l'une des revendications 1 à 8, **caractérisé en ce que** la séparation est effectuée en phase gazeuse.

11. Procédé selon l'une des revendications 1 à 10, **caractérisé en ce que** la charge est issue de la distillation atmosphérique.

12. Procédé selon l'une des revendications 1 à 10, **caractérisé en ce que** la charge est issue d'une unité de réformage, telle qu'un réformat léger.

13. Procédé selon l'une des revendications 1 à 10, **caractérisé en ce que** la charge est issue d'une unité de conversion, telle qu'un naphta d'hydrocraquage.

14. Procédé selon l'une des revendications 1 à 13, **caractérisé en ce que** les effluents respectivement riches en paraffines linéaires et en paraffines monobranchées sont envoyés vers au moins un réacteur d'hydro-isomérisation et l'effluent riche en paraffines multibranchées et éventuellement en composés naphténiques et/ou aromatiques est envoyé au pool essence.

## Claims

1. A process for separating straight chain, mono-branched and multi-branched paraffins from a feed constituted by a light cut comprising C5-C8, C5-C7, C6-C8, C7-C8, C6-C7, C7 or C8 paraffinic hydrocarbons, **characterized in that** the totality of said feed and an eluant are passed alternately and co-currently into a sole separation zone containing at least one adsorbent bed, whereby said adsorbent is selected from natural and synthetic adsorbents capable of separating straight chain paraffins, mono-branched paraffins and multi-branched paraffins and whereby said eluant consists in a compound that is not adsorbable by the adsorbent in the separation zone and **in that** the straight chain paraffins, mono-branched paraffins and multi-branched paraffins are separated in the form of three elution effluents, exiting successively from said separation zone.

2. A process according to claim 1, **characterized in that** the feed further comprises naphthenic and/or aromatic compounds, which are separated with the mono-branched paraffins.

3. A process according to claim 1 or 2, **characterized in that** the feed comprises the C5 cut and isopentane from this cut is separated with the mono-branched paraffins.

4. A process according to one of claims 1 to 3, **characterized in that** the feed comprises the C5 and the process further comprises a preliminary step in which isopentane contained in this cut is separated upstream of the separation zone.

5. A process according to any one of claims 1 to 4, **characterized in that** the feed comprises the C5 cut and isopentane contained in this cut is extracted from at least one of the effluents leaving the process.

6. A process according to claim 5, **characterized in that** the feed comprises the C5 cut and isopentane contained in this cut is extracted from the effluent which is rich in mono-branched paraffins leaving the process.

7. A process according to any one of claims 1 to 6, **characterized in that** at least one adsorbent selected from the group formed by silicalite, ZSM-5 zeolite, ZSM-11 zeolite, ZSM-23 zeolite and ferrierite is used.

8. A process according to claim 7, **characterized in that** said adsorbent also comprises a 5A zeolite.

9. A process according to any one of claims 1 to 8, **characterized in that** separation is carried out in the liquid phase.

10. A process according to any one of claims 1 to 8, **characterized in that** separation is carried out in the gas phase.

11. A process according to any one of claims 1 to 10, **characterized in that** the feed originates from atmospheric distillation.

12. A process according to any one of claims 1 to 10, **characterized in that** the feed originates from a reforming unit, for example a light reformate.

13. A process according to any one of claims 1 to 10, **characterized in that** the feed originates from a conversion unit, for example a hydrocracking naphtha.

14. A process according to any one of claims 1 to 13, **characterized in that** the effluents which are respectively rich in straight chain paraffins and in mono-branched paraffins are sent to at least one hydro-isomerisation reactor and the effluent which is rich in multi-branched paraffins and possibly in naphthenic and/or aromatic compounds is sent to the gasoline pool.

## Patentansprüche

1. Verfahren zur Trennung linearer einfach verzweigter und mehrfach verzweigter Paraffine aus einer aus einer leichten Fraktion gebildeten Charge, welche paraffinische Kohlenwasserstoffe C5-C8, C5-C7, C6-C8, C7-C8, C6-C7, C7 oder C8 umfasst, **dadurch gekennzeichnet, dass** man abwechselnd und im Gleichstrom in einer einzigen wenigstens ein Adsorberbett enthaltenden Trennzone die Gesamtheit dieser Charge und ein Eluierungsmittel strömen lässt, wobei der Adsorber gewählt ist aus den natürlichen oder synthetischen Adsorbern, die in der Lage sind, die linearen Paraffine, die einfach verzweigten Paraffine und die mehrfach verzweigten Paraffine zu trennen, und dieses Eluierungsmittel aus einer durch den Adsorber der Trennzone nicht adsorbierbaren Verbindung besteht, und dass man die linearen Paraffine, die einfach verzweigten Paraffine und die mehrfach verzweigten Paraffine in Form von drei Eluierungsströmen trennt, die nacheinander aus dieser Trennzone austreten.

2. Verfahren nach Anspruch 1, **dadurch gekennzeichnet, dass** die Charge im Übrigen naphtenische und/oder aromatische Kohlenwasserstoffe umfasst, die mit dem an mehrfach verzweigten Paraffinen reichen Abstrom abgetrennt werden.

3. Verfahren nach Anspruch 1 oder 2, **dadurch gekennzeichnet, dass** die Charge die C5-Fraktion und das Isopentan umfasst, das aus dieser Fraktion mit den einfach verzweigten Paraffinen abgetrennt wird.

4. Verfahren nach einem der Ansprüche 1 bis 3, **dadurch gekennzeichnet, dass** die Charge die C5-Fraktion umfasst, wobei das Verfahren im Übrigen eine Vorstufe aufweist, in der man das in dieser Fraktion vor der Trennzone enthaltene Isopentan abtrennt.

5. Verfahren nach einem der Ansprüche 1 bis 4, **dadurch gekennzeichnet, dass** die Charge die C5-Fraktion umfasst und das in dieser Fraktion enthaltene Isopentan über wenigstens einen der aus dem Verfahren austretenden Abströme abgezogen wird.

6. Verfahren nach Anspruch 5, **dadurch gekennzeichnet, dass** die Charge die C5-Fraktion umfasst und dass das in dieser Fraktion enthaltene Isopentan über den Abstrom abgezogen wird, der reich an aus dem Verfahren austretenden einfach verzweigten Paraffinen ist.

7. Verfahren nach einem der Ansprüche 1 bis 6, **dadurch gekennzeichnet, dass** man wenigstens einen Adsorber verwendet, der gewählt ist aus der Gruppe, die gebildet ist durch das Silikalit, den ZSM-5-Zeolithen, den ZSM-11-Zeolithen, den ZSM-23-Zeolithen und das Ferrierit.

8. Verfahren nach Anspruch 7, **dadurch gekennzeichnet, dass** dieser Adsorber im Übrigen einen 5A-Zeolithen umfasst.

9. Verfahren nach einem der Ansprüche 1 bis 8, **dadurch gekennzeichnet, dass** die Trennung in flüssiger Phase vorgenommen wird.

10. Verfahren nach einem der Ansprüche 1 bis 8, **dadurch gekennzeichnet, dass** die Trennung in gasförmiger Phase vorgenommen wird.

11. Verfahren nach einem der Ansprüche 1 bis 10, **dadurch gekennzeichnet, dass** die Charge aus der atmosphärischen Destillation stammt.

12. Verfahren nach einem der Ansprüche 1 bis 10, **dadurch gekennzeichnet, dass** die Charge aus einer Reformierungseinheit stammt, beispielsweise ein leichtes Reformat ist.

13. Verfahren nach einem der Ansprüche 1 bis 10, **dadurch gekennzeichnet, dass** die Charge aus einer Umwandlungseinheit stammt, beispielsweise ein Hydrocracknaphta ist.

14. Verfahren nach einem der Ansprüche 1 bis 13, **dadurch gekennzeichnet, dass** die an linearen Paraffinen und einfach verzweigten Paraffinen jeweils reichen Abströme gegen wenigstens einen Hydroisomerierungsreaktor geleitet werden und der an mehrfach verzweigten Paraffinen reiche und gegebenenfalls an naphtenischen und/oder aromatischen Verbindungen reiche Abstrom zum Benzinpool geleitet wird.
